# EUROPÄISCHE PATENTANMELDUNG

(11) **EP 4 759 794 A1**
(43) Veröffentlichungstag der Anmeldung: **17.06.2026**
(21) Anmeldenummer: 24218937.1
(22) Anmeldetag: 11.12.2024
(51) Int. Cl.: C07C 67/00, C07C 69/54, C07C 67/54, C08J 11/12

(54) **VERFAHREN ZUR ISOLIERUNG VON ALKYL(METH)ACRYLATEN**

(71) Anmelder: Röhm GmbH, 64295 Darmstadt (DE)
(72) Erfinder: KRILL, Steffen, 64367 Mühltal (DE); PÖFFEL, Wolfgang, 64665 Alsbach-Hähnlein (DE); VORHOLZ, Johannes, 63755 Alzenau (DE); KLASOVSKY, Florian, 45721 Haltern am See (DE)
(74) Vertreter: Röhm Patent Association

(57) **Zusammenfassung**

Die vorliegende Erfindung betrifft ein neues Verfahren zur Isolierung von Alkyl(meth)acrylaten, insbesondere von Methyl(meth)acrylaten. In diesem Verfahren wird zunächst eine Polymerzusammensetzung, die Poly(alkyl(meth)acrylat) enthält, thermisch gespalten unter Erhalt von mindestens einem Alkyl(meth)acrylat, mindestens einem weiteren Alkylester und mindestens einem Benzolderivat. Diese Mischung wird anschließend kondensiert und mit einem Strom eines Alkyl(meth)acrylat-Herstellverfahrens gemischt. Durch Destillation werden hochreine Alkyl(meth)acrylate isoliert.

## Beschreibung

### Gebiet der Erfindung

Die vorliegende Erfindung betrifft ein neues Verfahren zur Isolierung von Alkyl(meth)acrylaten, insbesondere von Methyl(meth)acrylaten. In diesem Verfahren wird zunächst eine Polymerzusammensetzung, die Poly(alkyl(meth)acrylat) enthält, thermisch gespalten unter Erhalt von mindestens einem Alkyl(meth)acrylat, mindestens einem weiteren Alkylester und mindestens einem Benzolderivat. Diese Mischung wird anschließend kondensiert und mit einem Strom eines Alkyl(meth)acrylat-Herstellverfahrens gemischt. Durch Destillation werden hochreine Alkyl(meth)acrylate isoliert.

### Stand der Technik

Alkyl(meth)acrylate, insbesondere Methylmethacrylat (MMA), werden heute mittels diverser Verfahren, die von C₂-, C₃- oder C₄-Bausteinen ausgehen, hergestellt.

In einem dieser Verfahren (C₄-Verfahren) wird MMA durch Oxidation von Isobutylen oder tert-Butanol mit Luftsauerstoff in der Gasphase an einem heterogenen Katalysator zu Methacrolein (MAL) und anschließender oxidativer Veresterungsreaktion von Methacrolein unter Verwendung von Methanol erhalten. Diese von ASAHI entwickelte Verfahren ist unter anderem in den Druckschriften US 5,969,178 und US 7,012,039 beschrieben.

Ein weiteres, weltweit verbreitetes, kommerzielles Verfahren basiert auf Aceton als Grundstoff und wird meist als C₃-Verfahren oder ACH-Sulfo-Prozess bezeichnet. Hierbei wird Aceton mit Blausäure (HCN) zum zentralen Zwischenprodukt Acetoncyanhydrin (ACH) umgesetzt. Dieses Zwischenprodukt wird isoliert und für die folgenden Prozessschritte zur Herstellung von Methacrylsäure (MAS) und MMA eingesetzt. Ein solcher Prozess ist beispielsweise in US 4,529,816 beschrieben.

Die WO 2014/170223 beschreibt ein sehr effizientes Verfahren, bei dem aus C₂-Fraktionen in einer ersten Stufe Propionaldehyd gewonnen wird und dieses in einer zweiten Stufe mit Formaldehyd zu Methacrolein umgesetzt wird. Dieses wiederum kann darauf in Anwesenheit von speziellen Metall- oder Metalloxidkatalysatoren und Methanol oxidativ zu MMA verestert werden. Dieses Verfahren wird auch als C₂-Verfahren bezeichnet.

MMA wird insbesondere zur Herstellung von Polymethylmethacrylat (PMMA) eingesetzt, welches sich durch hervorragende optische (Acrylglas) und andere physikalische Eigenschaften auszeichnet. Seine Einsatzgebiete umfassen Verglasungen und Fassaden in der Architektur, Beleuchtungselemente und Fahrzeugrückleuchten im Automobilbereich, Flugzeugfenster, Dekorations-, Design- oder Möbelelemente, Flachbildschirme und Displays, Lärmschutzwände und Lichtwerbungen.

Bei der Verarbeitung von PMMA fallen Produktionsabfälle, sogenannte Post Industrial-Abfälle, an. Darüber hinaus enthalten auch sogenannte Post Consumer-Abfälle, wie Elektro- und Altgeräteschrott, PMMA, häufig neben anderen Kunststoffen und Additiven. Der Anteil an MMA-Baueinheiten in den in den Abfällen enthaltenen PMMA-Polymeren ist unterschiedlich und kann von 75 % bis über 99 % liegen.

PMMA, sowohl als Post Industrial-Abfall, als auch als Post Consumer-Abfall, kann grundsätzlich in seine Monomere depolymerisiert und als sogenanntes Recycling-MMA wiederverwendet werden. Zur Depolymerisation sind im Stand der Technik verschiedene Verfahren, wie Depolymerisation in Metallbädern, Drehrohröfen, Wirbelschichtreaktoren oder Extrudern, beschrieben.

Beispielsweise beschreiben die Druckschriften DE 642289 C, US 2,030,901, DE 3146194 A1, EP 3 635 043 und US 2,470,361 die thermische Depolymerisation von PMMA, teilweise mit nachfolgender Aufreinigung der erhaltenen Monomere, beispielsweise mittels Destillation.

Thermisch-katalytische Depolymerisationen von PMMA mit anschließender Aufreinigung der erhaltenen Monomere sind beispielsweise in US 2,858,255, DE 2132716, WO 2019/003253, DE 19729065 und EP 2 895 576 beschrieben.

Ebenso bekannt ist die Depolymerisation von PMMA, teilweise zusammen mit anderen Polymeren, in einer Wirbelschicht. Dies ist beispielsweise in US 5,663,420, WO 2000/017149 und US 8,304,573 beschrieben.

US 3,494,958 beschreibt ein thermisches Depolymerisationsverfahren für PMMA. Das erhaltene Monomer kann anschließend mittels Destillation aufgereinigt werden. Die US 3,494,958 beschreibt, dass die Aufreinigung beispielsweise analog zur Aufreinigung in einem ACH-Prozess erfolgen kann.

In den vorstehend beschriebenen Verfahren ist die Ausbeute an Monomeren teilweise nicht ausreichend. Zudem erzielen die im Stand der Technik beschriebenen Verfahren recycelte Alkyl(meth)acrylate, deren Reinheit nicht ausreichend ist zur Herstellung von Poly(alkyl(meth)acrylaten) ausreichender Qualität. Dies ist insbesondere der Fall, wenn in den Depolymerisationsprozessen Poly(methylmethacrylat) eingesetzt wird, das einen großen Anteil an Additiven, wie Schlagzähmodifizierer oder Pigmente und/oder andere Polymere enthält. Zudem ist das global warming potential (GWP) der vorgeschlagenen Verfahren nicht ausreichend.

### Aufgabe

Es bestand somit Bedarf, ein verbessertes Verfahren zur Isolierung von Alkyl(meth)acrylaten, insbesondere von Methylmethacrylat, aus einem Depolymerisationsprozess bereitzustellen, das die Nachteile der im Stand der Technik beschriebenen Verfahren nicht oder nur in vermindertem Maße aufweist. Insbesondere soll das Verfahren ein geringeres global warming potential (GWP), also einen geringeren Ausstoß an klimawirksamen Abfallstoffen wie CO₂, aufweisen.

### Lösung

Gelöst wird diese Aufgabe durch ein Verfahren zur Isolierung von Alkyl(meth)acrylaten umfassend die folgenden Schritte a) bis e):
a) thermische Spaltung von mindestens einer Polymerzusammensetzung, die mindestens ein Poly(alkyl(meth)acrylat) enthält, unter Erhalt eines ersten Gasstroms, der mindestens ein Alkyl(meth)acrylat, mindestens einen weiteren Alkylester und mindestens ein Benzolderivat enthält,
b) Kondensation des in Schritt a) erhaltenen ersten Gasstroms unter Erhalt eines flüssigen ersten Stroms, der das mindestens eine Alkyl(meth)acrylat, den mindestens einen weiteren Alkylester und mindestens ein Benzolderivat enthält,
c) Mischen des in Schritt b) erhaltenen flüssigen ersten Stroms mit einem weiteren Strom, der Alkyl(meth)acrylat enthält, wobei der weitere Strom Teil eines Alkyl(meth)acrylat-Herstellverfahrens ist, unter Erhalt eines Mischstroms, der den flüssigen ersten Strom und den weiteren Strom umfasst,
d) Destillation des in Schritt c) erhaltenen Mischstroms unter Erhalt eines Sumpfstroms, der Alkyl(meth)acrylat enthält, und eines Kopfstroms, der den mindestens einen weiteren Alkylester enthält,
e) zumindest teilweise Hydrolyse des in Schritt d) erhaltenen Kopfstroms, wobei der mindestens eine weitere Alkylester zumindest teilweise hydrolysiert unter Erhalt einer Alkylsäure.

Es wurde überraschend gefunden, dass durch das erfindungsgemäße Verfahren Alkyl(meth)acrylate, insbesondere Methylmethacrylat, mit einer Reinheit von >99,5 % erhalten werden.

Insbesondere können durch das erfindungsgemäße Verfahren überraschenderweise Nebenprodukte wie weitere Alkylester und Benzolderivate besonders effizient entfernt werden, selbst wenn sich deren Normalsiedetemperatur nur geringfügig, beispielsweise nur um +/- 20 K (Kelvin), vorzugsweise um +/- 1 K, besonders bevorzugt um +/- 0,6 K, von der Normalsiedetemperatur des Alkyl(meth)acrylats unterscheidet. Dies ist insbesondere durch Verfahrensschritt e) bedingt.

Auch Additive und Kettenregler, die in der Polymerzusammensetzung enthalten sein können, und die aus ihnen bei der thermischen Spaltung der Polymerzusammensetzungen erhaltenen Produkte wie Mercaptane lassen sich einfach und kostengünstig durch das erfindungsgemäße Verfahren abtrennen. Beispielsweise enthalten die im erfindungsgemäßen Verfahren erhaltenen Alkyl(meth)acrylate maximal 0,5 % Nebenprodukte. Aufgrund des erfindungsgemäßen Verfahrens und der dadurch möglichen effizienten Abtrennung der Nebenprodukte, weisen die erfindungsgemäß hergestellten Alkyl(meth)acrylate, trotz der in der Polymerzusammensetzung enthaltenen schwefelhaltigen Verbindungen, keinen oder nur einen sehr geringen Geruch auf.

Das erfindungsgemäße Verfahren weist zudem ein geringeres global warming potential (GWP), insbesondere einen geringen COz-Ausstoß auf. Dies wird insbesondere dadurch erzielt, dass das erfindungsgemäße Verfahren auch die Verwendung von Polymerzusammensetzungen mit einem relativ hohen Anteil an Additiven und sonstigen Verunreinigungen erlaubt. Zudem können die bei der Depolymerisation erhaltenen Nebenprodukte nach ihrer Abtrennung weiterverwertet werden, sodass das global warming potential (GWP) weiter reduziert wird.

Es wurde weiterhin überraschend gefunden, dass in einer bevorzugten Ausführungsform des erfindungsgemäßen Verfahrens die zumindest teilweise Hydrolyse in Schritt e) innerhalb des Alkyl(meth)acrylat-Herstellverfahrens stattfinden kann, sodass keine zusätzlichen Apparaturen zur Hydrolyse bereitgestellt werden müssen.

Durch das Mischen des erhaltenen flüssigen ersten Stroms mit dem weiteren Strom, der Teil eines Alkyl(meth)acrylat-Herstellverfahrens ist, und die in einer bevorzugten Ausführungsform des erfindungsgemäßen Verfahrens innerhalb des Alkyl(meth)acrylat-Herstellverfahrens durchgeführte Hydrolyse, können bestehende Alkyl(meth)acrylat-Anlagen zur Aufreinigung des flüssigen ersten Stroms eingesetzt werden. Dies reduziert den apparativen Aufwand des erfindungsgemäßen Verfahrens enorm.

Das erfindungsgemäße Verfahren ist somit den bisher bekannten überlegen, da es der Anforderung gerecht wird, sich selbst in der Kreislaufkette wiederverwenden zu können (circular economy).

Zudem können die bei der Depolymerisation erhaltenen Nebenprodukte nach ihrer Abtrennung weiterverwertet werden, sodass der Carbon Footprint und damit das global warming potential (GWP) weiter reduziert wird.

Nachfolgend wird das erfindungsgemäße Verfahren näher beschrieben.

In Schritt a) des erfindungsgemäßen Verfahrens wird mindestens eine Polymerzusammensetzung thermisch gespalten unter Erhalt eines ersten Gasstroms. Die mindestens eine Polymerzusammensetzung enthält mindestens ein Poly(alkyl(meth)acrylat). Der Gasstrom enthält mindestens ein Alkyl(meth)acrylat, mindestens einen weiteren Alkylester und mindestens ein Benzolderivat.

Der Begriff "mindestens eine Polymerzusammensetzung" bedeutet im Rahmen der vorliegenden Erfindung sowohl genau eine Polymerzusammensetzung als auch eine Mischung aus zwei oder mehreren Polymerzusammensetzungen. Eine Mischung aus zwei oder mehreren Polymerzusammensetzungen ist erfindungsgemäß bevorzugt.

Die mindestens eine Polymerzusammensetzung enthält mindestens ein Poly(alkyl(meth)acrylat).

"Mindestens ein Poly(alkyl(meth)acrylat)" bedeutet im Rahmen der vorliegenden Erfindung sowohl genau ein Poly(alkyl(meth)acrylat) als auch eine Mischung aus zwei oder mehreren Poly(alkyl(meth)acrylaten). Unter "Poly(alkyl(meth)acrylaten)" werden im Rahmen der vorliegenden Erfindung Polymere und Copolymere von Alkyl(meth)acrylaten verstanden.

Copolymere von Alkyl(meth)acrylaten sind beispielsweise Copolymere von Alkyl(meth)acrylaten mit 1-Alkenen, anderen Alkyl(meth)acrylaten, (Meth)acrylsäure, Styrol, Polyestern und/oder Polyurethan(meth)acrylaten.

1-Alkene, die mit Alkyl(meth)acrylaten copolymerisieren können, sind als solche bekannt und beispielsweise Hexen-1, Hepten-1, Vinylcyclohexan, 3,3-Dimethyl-1-propen, 3-Methyl-1-diisobutylen und 4-Methylpenten-1.

Unter dem Begriff "Styrol" wird im Rahmen der vorliegenden Erfindung nicht nur Styrol als solches verstanden, sondern auch substituierte Styrole wie beispielsweise α-Methylstyrol, α-Ethylstyrol, Vinyltoluol, p-Methylstyrol, Monochlorstyrole, Dichlorstyrole und Tribromstyrole.

Geeignete Polyester sind als solche bekannt und vorzugsweise via Polykondensation oder ringöffnende Polymerisation erhältlich.

Unter "Polyurethan(meth)acrylaten" werden im Rahmen der vorliegenden Erfindung (Meth)acrylate verstanden, die über Urethangruppen miteinander verknüpft sind. Sie sind durch Umsetzung von Hydroxyalkyl(meth)acrylaten mit Polyisocyanaten und Polyoxyalkylenen, die mindestens zwei Hydroxyfunktionalitäten aufweisen, erhältlich. Anstelle von Hydroxyalkyl(meth)acrylaten können auch Ester der (Meth)acrylsäure mit Oxiranen, wie beispielsweise Ethylenoxid oder Propylenoxid, oder entsprechende Oligooxirane bzw. Polyoxirane verwendet werden. Geeignete Polyurethan(meth)acrylate sind als solche bekannt.

Die mindestens eine Polymerzusammensetzung kann beispielsweise aus Produktionsabfällen stammen. Dann handelt es sich bei der Polymerzusammensetzung üblicherweise um sogenannte Post Industrial-Abfälle, wie beispielsweise Angüsse, Anfahrklumpen bei der Extrusion, Stäube, Späne, anpolymerisierte Polymersirupe, Randabschnitte, Verschnitte bei Platten, Ausschuss bei Platten, Folien, Blöcke, Halbzeuge, Fehlfabrikationen von Formteilen oder Abfälle beim Spritzgießen.

Ebenso ist es möglich, dass die mindestens eine Polymerzusammensetzung aus sogenannten Post Consumer-Abfällen stammt. Dann handelt es sich üblicherweise um Abfälle von beispielsweise Elektro- und Altgeräteschrott, Gewächshäuser, Messebauten, Shop-Fittinge oder Lichtwerbung.

Die mindestens eine Polymerzusammensetzung enthält daher üblicherweise zumindest eine weitere Komponente. Die zumindest eine weitere Komponente ist beispielsweise ausgewählt aus der Gruppe bestehend aus von dem Poly(alkyl(meth)acrylat) verschiedenen Polymeren, Pigmenten, Farbstoffen, Füllstoffen, Hilfsstoffen, Reglern, Initiatoren, Schlagzähmachern, Trennmitteln und UV-Additiven.

Bevorzugt ist daher auch ein Verfahren, bei dem die Polymerzusammensetzung in Schritt a) zumindest eine weitere Komponente, ausgewählt aus der Gruppe bestehend aus von Poly(alkyl(meth)acrylat) verschiedenen Polymeren, Pigmenten, Farbstoffen, Füllstoffen, Hilfsstoffen, Reglern, Initiatoren, Schlagzähmachern, Trennmitteln und UV-Additiven, enthält.

Als von Poly(alkyl(meth)acrylat) verschiedene Polymere kommen insbesondere solche Polymere in Betracht, die üblicherweise als Blend mit Poly(alkyl(meth)acrylat) eingesetzt werden können. Hierzu zählen beispielsweise Polyethylen, Polyvinylchlorid, Polystyrol, Polyamide und Biopolymere wie α-Polysaccharide (Stärke), β-Polysaccharide (Cellulose, Chitin) Lignin und Polylactid.

Pigmente sind beispielsweise weiße, rote, blaue, grüne und/oder gelbe anorganische Pigmente. Besonders bevorzugt sind weiße, anorganische Pigmente wie Titandioxid.

Farbstoffe sind beispielsweise dem Fachmann bekannte organische Farbstoffe.

Typische Füllstoffe sind insbesondere mineralische Füllstoffe. Mineralische Füllstoffe sind vorzugsweise ausgewählt aus der Gruppe bestehend aus Calciumcarbonat, Bariumsulfat, Quarz, Quarzmehl, gefällten Kieselsäuren, pyrogenen Kieselsäuren, Korunde, Glasperlen und Cristobaliten.

Typische Hilfsstoffe sind als solche bekannt und beispielsweise ausgewählt aus der Gruppe bestehend aus Weichmachern, Paraffinen und/oder Inhibitoren.

Als Weichmacher werden vorzugsweise Ester, Polyole, Öle, niedermolekulare Polyether oder Phthalate eingesetzt.

Paraffine, die in der Polymerzusammensetzung enthalten sein können, sind als solche bekannt. Es können beispielsweise mehrere Paraffine mit unterschiedlichen Schmelzpunkten in unterschiedlichen Konzentrationen enthalten sein.

Aus der Gruppe der Inhibitoren werden vorzugsweise substituierte Phenole, Hydrochinonderivate, Phosphine und/oder Phosphite eingesetzt.

Als Regler kommen insbesondere aus der radikalischen Polymerisation bekannte Verbindungen, die die Kettenlängen regeln, in Betracht. Üblicherweise umfassen Kettenregler Mercaptane wie n-Dodecylmercaptan, aber auch mehrwertige Mercaptoverbindungen wie Pentaerythroltetrathioglycolat.

Initiatoren sind als solche ebenfalls bekannt und beispielsweise ausgewählt aus der Gruppe bestehend aus Peroxiden, Azoverbindungen, Persulfaten und Mischungen daraus.

Schlagzähmacher sind als solche bekannt und beispielsweise Polymerpartikel, die ein Elastomer enthalten.

Als Trennmittel kommen insbesondere langkettige Wachssäuren, wie Stearinsäure, Palmitinsäure oder Laurinsäure, sowie einwertige Fett- oder Wachsalkohole, wie beispielsweise Diethylenglycolmonopropylether, in Betracht.

Als UV-Additive kommen insbesondere UV-Stabilisatoren in Frage. Vorzugsweise werden die UV-Stabilisatoren ausgewählt aus der Gruppe bestehend aus Benzophenonderivaten, Benzotriazolderivaten, Thioxanthonatderivaten, Piperidinolcarbonsäureesterderivaten und Zimtsäureesterderivaten.

Die thermische Spaltung der mindestens einen Polymerzusammensetzung kann nach dem Fachmann bekannten Methoden erfolgen.

Die thermische Spaltung kann in dem Fachmann bekannten Reaktoren für thermische Spaltungen durchgeführt werden. Beispielsweise kann die thermische Spaltung in einem Pyrolysereaktor, in einem Extruder, in einem Drehrohrofen, einer Wirbelschichtpyrolyse, in einem Metallbad und/oder als trockene Destillation durchgeführt werden.

Die Polymerzusammensetzung kann bei der thermischen Spaltung in fester oder flüssiger Form vorliegen. Liegt die Polymerzusammensetzung in fester Form vor, so kann sie als reiner Feststoff vorliegen. Ebenso ist es möglich, dass die Polymerzusammensetzung in fester Form in einem Medium dispergiert vorliegt. Das Medium, in dem die Polymerzusammensetzung dispergiert vorliegen kann, kann beispielsweise ein Feststoff, wie Quarz, Metallspäne oder Kieselgur, sein. Ebenso ist es möglich, dass das Medium ein Gas ist wie beispielsweise Stickstoff oder eine Flüssigkeit, wie beispielsweise Wasser oder ein Kohlenwasserstoff. Es ist möglich, dass das Medium bei Raumtemperatur eine Flüssigkeit ist, bei den Bedingungen der thermischen Spaltung in Schritt a) allerdings als Gas vorliegt.

Die Polymerzusammensetzung liegt beispielsweise in flüssiger (geschmolzener) form vor, wenn die thermische Spaltung in einem Extruder erfolgt.

Die Polymerzusammensetzung kann, insbesondere wenn sie in fester Form vorliegt, vor der thermischen Spaltung mechanisch zerkleinert werden, beispielsweise auf eine durchschnittliche Teilchengrößenverteilung von unter 6 mm (Qr, d50) bevorzugt unter 1,5 mm (Qr, d50), beispielsweise auf eine Teilchengrößenverteilung im Bereich von 0,1 mm (Qr, d50) bis 6 mm (Qr, d50). Vorzugsweise wird die Teilchengrößenverteilung mittels Laserdiffraktometrie bestimmt.

Die Temperatur (T) bei der thermischen Spaltung in Schritt a) liegt beispielsweise in einem Bereich von 240 °C bis 800 °C, bevorzugt bei einer Temperatur im Bereich von 300 °C bis 500 °C, besonders bevorzugt bei einer Temperatur im Bereich von 325 °C bis 400 °C.

Der Druck bei der thermischen Spaltung in Schritt a) liegt beispielsweise im Bereich von 200 mbar bis 1000 bar, bevorzugt im Bereich von 400 mbar bis 500 bar.

Erfindungsgemäß bevorzugt ist daher auch ein Verfahren, bei dem die Temperatur bei der thermischen Spaltung in Schritt a) im Bereich von 240 °C bis 800 °C liegt und/oder der Druck bei der thermischen Spaltung im Bereich von 400 mbar bis 500 bar liegt.

Die thermische Spaltung in Schritt a) kann in Gegenwart eines Katalysators erfolgen. Für die thermische Spaltung geeignete Katalysatoren sind als solche bekannt und beispielsweise ausgewählt aus der Gruppe bestehend aus geschmolzenem Metall, Peroxiden, Kaliumsalzen wie beispielsweise Kaliumacetat, und Alkalimetallpersulfaten, wie beispielsweise Kaliummonopersulfat. Als geschmolzenes Metall eignen sich insbesondere Blei und/oder eutektische Blei/Zinn Mischungen.

Erfolgt die thermische Spaltung in Schritt a) in Gegenwart eines Katalysators, so wird die Spaltung in Schritt a) auch als thermisch katalytische Spaltung bezeichnet.

Erfindungsgemäß bevorzugt ist daher auch ein Verfahren, bei dem die thermische Spaltung in Schritt a) eine thermisch katalytische Spaltung ist.

Bei der thermischen Spaltung in Schritt a) wird das Poly(alkyl(meth)acrylat) gespalten. Diese Spaltung wird auch als Depolymerisation bezeichnet. Depolymerisationen als solche sind dem Fachmann bekannt. Bei Depolymerisationen wird ein Polymer in seine Monomer- und Oligomereinheiten gespalten.

Bei der thermischen Spaltung in Schritt a) wird also das Poly(alkyl(meth)acrylat) in seine Monomer- und Oligomereinheiten gespalten. Wie vorstehend ausgeführt, ist das Poly(alkyl(meth)acrylat) ein Polymer oder Copolymer von mindestens einem Alkyl(meth)acrylat. Bei der thermischen Spaltung bildet sich daher mindestens ein Alkyl(meth)acrylat.

Bei der thermischen Spaltung der Polymerzusammensetzung bildet sich zudem mindestens ein weiterer Alkylester. "Mindestens ein weiterer Alkylester" bedeutet im Rahmen der vorliegenden Erfindung sowohl genau ein weiterer Alkylester als auch eine Mischung aus zwei oder mehreren weiteren Alkylestern. Eine Mischung aus zwei oder mehreren weiteren Alkylestern ist erfindungsgemäß bevorzugt.

Darüber hinaus bildet sich bei der thermischen Spaltung üblicherweise mindestens ein Benzolderivat. Das mindestens eine Benzolderivat bildet sich beispielsweise bei der thermischen Spaltung der Polymerzusammensetzung aus von dem Poly(alkyl(meth)acrylat) verschiedenen Polymeren wie Polystyrol. "Mindestens ein Benzolderivat" bedeutet im Rahmen der vorliegenden Erfindung sowohl genau ein Benzolderivat als auch eine Mischung aus zwei oder mehreren Benzolderivaten. Eine Mischung aus zwei oder mehreren Benzolderivaten ist erfindungsgemäß bevorzugt.

Der erste Gasstrom enthält daher das bei der thermischen Spaltung gebildeten mindestens eine Alkyl(meth)acrylat, den mindestens einen weiteren Alkylester und das mindestens eine Benzolderivat.

Das mindestens eine Alkyl(meth)acrylat ist, wie vorstehend ausgeführt, von dem Poly(alkyl(meth)acrylat) abgeleitet.

"Mindestens ein Alkyl(meth)acrylat" bedeutet im Rahmen der vorliegenden Erfindung sowohl genau ein Alkyl(meth)acrylat als auch eine Mischung aus zwei oder mehreren Alkyl(meth)acrylaten.

Unter "Alkyl(meth)acrylaten" werden im Rahmen der vorliegenden Erfindung Alkylester von (Meth)acrylsäure verstanden, die 1 bis 18, bevorzugt 1 bis 12, insbesondere bevorzugt 1 bis 4, Kohlenstoffatome im Alkylrest aufweisen. Der Alkylrest kann linear, zyklisch und/oder verzweigt sein. Darüber hinaus kann er aromatische Reste aufweisen. Der Alkylrest kann zudem Heteroatome innerhalb des Alkylrests aufweisen und/oder mit Heteroatomen substituiert sein, wie dies beispielsweise bei Hydroxypropyl(meth)acrylat und/oder Hydroxyethyl(meth)acrylat der Fall ist. Beispielsweise sind erfindungsgemäße Alkyl(meth)acrylate ausgewählt aus der Gruppe bestehend aus Methyl(meth)acrylat, Ethyl(meth)acrylat, 1-Methyl-Ethyl(meth)acrylat, Propyl(meth)acrylat, n-Butyl(meth)acrylat, Isobutyl(meth)acrylat, 2-Ethylhexyl(meth)acrylat, Isopentyl(meth)acrylat, Cyclohexyl(meth)acrylat, Tetrahydrofurfuryl(meth)acrylat, Allyl(meth)acrylat, Polyethylenglycol(meth)acrylat, Stearyl(meth)acrylat, Benzyl(meth)acrylat, Vinyl(meth)acrylat, Hydroxypropyl(meth)acrylat, Hydroxyethyl(meth)acrylat und Lauryl(meth)acrylat.

In einer weiteren Ausführungsform der vorliegenden Erfindung werden unter "Alkyl(meth)acrylaten" auch Polyethylenglycol(meth)acrylate mit einem gewichtsmittleren Molekulargewicht Mw im Bereich von 250 g/mol bis 10.000 g/mol verstanden.

Der Begriff "(Meth)acrylsäure" umfasst im Rahmen der vorliegenden Erfindung sowohl Acrylsäure als auch Methacrylsäure. Der Begriff "(Meth)acrylate" umfasst im Rahmen der vorliegenden Erfindung sowohl Acrylate als auch Methacrylate.

Unter "Alkyl(meth)acrylaten" werden im Rahmen der vorliegenden Erfindung daher sowohl Alkylmethacrylate als auch Alkylacrylate verstanden. Alkylmethacrylate sind erfindungsgemäß bevorzugt. Das mindestens eine Alkyl(meth)acrylat ist beispielsweise ein C₁-C₁₈-Alkyl(meth)acrylat, bevorzugt ein C₁-C₁₂-Alkyl(meth)acrylat und besonders bevorzugt ein C₁-C₄-Alkyl(meth)acrylat. Erfindungsgemäß besonders bevorzugt umfasst das mindestens eine Alkyl(meth)acrylat Methyl(meth)acrylat.

Bevorzugt ist daher auch ein Verfahren, bei dem das in dem ersten Gasstrom in Schritt a) enthaltene mindestens eine Alkyl(meth)acrylat ausgewählt ist aus der Gruppe bestehend aus C₁- bis C₄-Alkyl(meth)acrylaten.

Das mindestens eine Alkyl(meth)acrylat weist beispielsweise eine Siedetemperatur bei Normaldruck im Bereich von 50 °C bis 300 °C, bevorzugt im Bereich von 80 °C bis 250 °C, auf.

Es versteht sich von selbst, dass der mindestens eine weitere Alkylester von dem mindestens einen Alkyl(meth)acrylat verschieden ist. Der mindestens eine weitere Alkylester umfasst vorzugsweise keine (Meth)acrylat-Einheit.

Beispielsweise ist der mindestens eine weitere Alkylester ausgewählt aus der Gruppe bestehend aus C₁-C₄-Alkylisobutyraten, C₁-C₄-Alkylpropionaten, C₁-C₄-Alkylpivalaten und Dicarbonsäurediestern.

Unter C₁-C₄-Alkylisobutyraten werden im Rahmen der vorliegenden Erfindung Alkylester der Isobuttersäure verstanden, die 1 bis 4 Kohlenstoffatome im Alkylrest aufweisen. Der Alkylrest kann linear oder verzweigt sein. Er kann zudem Heteroatome innerhalb des Alkylrests aufweisen und/oder mit Heteroatomen substituiert sein. Ein C₁-C₄-Alkylisobutyrat, das Heteroatome innerhalb des Alkylrests aufweist, ist beispielsweise Methyl-3-methoxyisobutyrat und Methyl-3-methoxyisobutyrat. Erfindungsgemäße C₁-C₄-Alkylisobutyrate sind beispielsweise ausgewählt aus der Gruppe bestehend aus Methylisobutyrat, Ethylisobutyrat, Methyl-2-methoxyisobutyrat und Methyl-3-methoxyisobutyrat.

Unter C₁-C₄-Alkylpropionaten werden im Rahmen der vorliegenden Erfindung Alkylester der Propionsäure verstanden, die 1 bis 4 Kohlenstoffatome im Alkylrest aufweisen. Der Alkylrest kann linear oder verzweigt sein. Er kann zudem Heteroatome innerhalb des Alkylrests aufweisen und/oder mit Heteroatomen substituiert sein. Erfindungsgemäße C₁-C₄-Alkylpropionate sind beispielsweise ausgewählt aus der Gruppe bestehend aus Methylpropionat und Ethylpropionat.

Unter C₁-C₄-Alkylpivalaten werden im Rahmen der vorliegenden Erfindung Alkylester der Pivalinsäure verstanden, die 1 bis 4 Kohlenstoffatome im Alkylrest aufweisen. Der Alkylrest kann linear oder verzweigt sein. Er kann zudem Heteroatome innerhalb des Alkylrests aufweisen und/oder mit Heteroatomen substituiert sein. Erfindungsgemäße C₁-C₄-Alkylpivalate sind beispielsweise ausgewählt aus der Gruppe bestehend aus Methylpivalat und Ethylpivalat.

Unter Dicarbonsäurediestern werden im Rahmen der vorliegenden Erfindung Alkylester von Dicarbonsäuren verstanden, die 1 bis 4 Kohlenstoffe im Alkylrest aufweisen. Ebenso werden unter Dicarbonsäurediestern Carbonsäureester von Diolen verstanden, bei denen beide Hydroxygruppen mit einer Carbonsäure verestert sind. Erfindungsgemäße Dicarbonsäurediester sind beispielsweise ausgewählt aus der Gruppe bestehend aus Propandisäure-dimethylester, Pentandisäuredimethylester, Hexandisäure-dimethylester und Heptandisäure-dimethylester.

Vorzugsweise ist der mindestens eine weitere Alkylester ausgewählt aus der Gruppe bestehend aus Methylpropionat, Ethylpropionat, Methylisobutyrat, Methylpivalat, Methyl-3-methoxyisobutyrat und Dicarbonsäurediestern.

Bevorzugt ist daher auch ein Verfahren, bei dem der mindestens eine weitere Alkylester ausgewählt ist aus der Gruppe bestehend aus Methylpropionat, Methylisobutyrat, Methylpivalat, Methyl-3-methoxyisobutyrat und Dicarbonsäurediestern.

Der mindestens eine weitere Alkylester weist beispielsweise eine Siedetemperatur bei Normaldruck im Bereich von 50 °C bis 200 °C, bevorzugt im Bereich von 75°C bis 150 °C, auf.

Erfindungsgemäß weiterhin bevorzugt unterscheidet sich der Siedepunkt bei Normaldruck von zumindest einem mindestens einem weiteren Alkylester von dem Siedepunkt bei Normaldruck von zumindest einem mindestens einem Alkyl(meth)acrylat um im Bereich von -20 °C bis +20 °C, bevorzugt um im Bereich von -1 °C bis +1 °C, besonders bevorzugt um im Bereich von -0,6 °C bis +0,6 °C.

Unter "Benzolderivat" werden im Rahmen der vorliegenden Erfindung von Benzol abgeleitete Verbindungen verstanden, bei denen mindestens ein H-Atom des Benzolrings substituiert wurde. Die Substitution kann durch einen Kohlenstoff-haltigen Rest und/oder Heteroatome erfolgen. Zu den Kohlenstoffhaltigen Reste zählen insbesondere Alkylreste, Allylreste und Vinylreste. Vorzugsweise ist das mindestens eine Benzolderivat ausgewählt aus der Gruppe bestehend aus Toluol, Trimethylbenzol und Styrol.

Unter "Trimethylbenzol" werden im Rahmen der vorliegenden Erfindung sämtliche Isomere von Trimethylbenzol verstanden, also 1,2,3-Trimethylbenzol, 1,2,4-Trimethylbenzol und 1,3,5-Trimethylbenzol.

Für Styrol als das mindestens eine Benzolderivat gelten die zuvor beschriebenen Ausführungen und Bevorzugungen für Styrol als Comonomer entsprechend.

Wie vorstehend ausgeführt wird der erste Gasstrom durch thermische Spaltung der mindestens einen Polymerzusammensetzung erhalten. Üblicherweise enthält der erste Gasstrom daher mindestens eine weitere Komponente. Es versteht sich von selbst, dass die mindestens eine weitere Komponente von dem mindestens einen Alkyl(meth)acrylat, dem mindestens einen weiteren Alkylester und dem mindestens einen Benzolderivat verschieden ist.

Die mindestens eine weitere Komponente bildet sich beispielsweise durch thermische Spaltung von Copolymeren von Alkyl(meth)acrylat und/oder durch thermische Spaltung der in der mindestens einen Polymerzusammensetzung gegebenenfalls enthaltenen zumindest einen weiteren Komponente. Darüber hinaus können sich bei der thermischen Spaltung auch Oligomere von Alkyl(meth)acrylat bilden, diese fallen im Rahmen der vorliegenden Erfindung ebenfalls unter den Begriff "mindestens eine weitere Komponente".

Beispielsweise ist die mindestens eine weitere Komponente ausgewählt aus der Gruppe bestehend aus (Meth)acrylsäure, schwefelhaltigen Verbindungen, Oligomeren, Dimeren, C₁-C₄-Alkylsäuren, Di(meth)acrylatdiestern, C₁-C₄-Alkoholen, Aldehyden und Ketonen, bevorzugt ist die weitere Komponente ausgewählt aus der Gruppe bestehend aus (Meth)acrylsäure, schwefelhaltigen Verbindungen, Oligomeren und Dimeren.

Bevorzugt ist daher auch ein Verfahren, bei dem der erste Gasstrom mindestens eine weitere Komponente, ausgewählt aus der Gruppe bestehend aus(Meth)acrylsäure, schwefelhaltigen Verbindungen, Oligomeren und Dimeren, enthält.

Schwefelhaltige Verbindungen leiten sich insbesondere von gegebenenfalls in der mindestens einen Polymerzusammensetzung enthaltenen schwefelhaltigen Reglern ab und sind insbesondere Mercaptane wie Dodecylmercaptan und mehrwertige Mercaptoverbindungen wie Pentaerythroltetrathoglycolat.

Unter Oligomeren werden im Rahmen der vorliegenden Erfindung insbesondere Oligomere des mindestens einen Alkyl(meth)acrylats, von (Meth)acrylsäure und von Mischungen aus diesen verstanden. Der Begriff "Oligomer" umfasst nicht nur höhere Oligomere, sondern auch niedere Oligomere, wie beispielsweise Trimere, Tetramere und Pentamere. Insbesondere umfasst der Begriff "Oligomer" ein Molekül, das aus drei bis zehn Einheiten aufgebaut ist, wobei die Einheiten aus dem mindestens einen Alkyl(meth)acrylat, (Meth)acrylsäure oder Mischungen daraus erhältlich sind.

Unter Dimeren werden im Rahmen der vorliegenden Erfindung insbesondere Dimere des mindestens einen Alkyl(meth)acrylats, von (Meth)acrylsäure und von Mischungen aus diesen verstanden.

Unter C₁-C₄-Alkylsäuren werden aliphatische Carbonsäuren mit 1 bis 4 Kohlenstoffatomen verstanden. Die C₁-C₄-Alkylsäuren können Verzweigungen aufweisen. Beispielsweise ist die C₁-C₄-Alkylsäure ausgewählt aus der Gruppe bestehend aus Ameisensäure, Essigsäure, Propionsäure, Buttersäure und Isobuttersäure.

Unter Di(meth)acrylatdiestern werden Ester von (Meth)acrylsäure mit einem Diol verstanden. Beispielsweise ist der Di(meth)acrylatdiester ausgewählt aus der Gruppe bestehend aus Ethylenglycoldi(meth)acrylat und Propylenglycoldi(meth)acrylat.

Unter C₁-C₄-Alkoholen werden Alkohole verstanden, die 1 bis 4 Kohlenstoffatome im Alkylrest aufweisen. Der Alkylrest kann linear oder verzweigt sein. Ebenso ist es möglich, dass er mit Heteroatomen substituiert ist. Beispiele für C₁-C₄-Alkohole sind Methanol, Ethanol, Ethylenglycol, n-Butanol und Isobutanol.

Aldehyde sind dem Fachmann als solche bekannt. Beispielsweise sind die Aldehyde ausgewählt aus der Gruppe bestehend aus Formaldehyd, Acetaldehyd, Methacrolein und Acrolein.

Unter Ketonen werden im Rahmen der vorliegenden Erfindung sowohl Monoketone wie beispielsweise Aceton als auch Diketone wie beispielsweise Diacetyl verstanden. Erfindungsgemäße Ketone sind beispielsweise ausgewählt aus der Gruppe bestehend aus Diacetyl, Aceton, Acetylaceton und Methylethylketon.

Beispielsweise enthält der bei der thermischen Spaltung erhaltene erste Gasstrom 60 bis 99 Gew.-%, bevorzugt 80 bis 98 Gew.-%, des mindestens einen Alkyl(meth)acrylats, jeweils bezogen auf das Gesamtgewicht des ersten Gasstroms.

Beispielsweise enthält der bei der thermischen Spaltung erhaltene erste Gasstrom 0,01 bis 5 Gew.-%, bevorzugt 0,1 bis 1 Gew.-%, des mindestens einen weiteren Alkylesters, jeweils bezogen auf das Gesamtgewicht des ersten Gasstroms.

Beispielsweise enthält der bei der thermischen Spaltung erhaltene erste Gasstrom 0,01 bis 5 Gew.-% , bevorzugt 0,05 bis 0,5 Gew.-%, des mindestens einen Benzolderivats, jeweils bezogen auf das Gesamtgewicht des ersten Gasstroms.

Beispielsweise enthält der bei der thermischen Spaltung erhaltene erste Gasstrom 0,01 bis 5 Gew.-%, bevorzugt 0,1 bis 3 Gew-%, der mindestens einen weiteren Komponente, jeweils bezogen auf das Gesamtgewicht des ersten Gasstroms.

In Schritt b) wird der in Schritt a) erhaltene erste Gasstrom unter Erhalt eines flüssigen ersten Stroms kondensiert. Der flüssige erste Strom enthält das mindestens eine Alkyl(meth)acrylat, den mindestens einen weiteren Alkylester und das mindestens eine Benzolderivat.

Der erste Gasstrom kann nach dem Fachmann bekannten Methoden kondensiert werden, beispielsweise kann die Kondensation an einem Kondensator erfolgen.

Beispielsweise wird der erste Gasstrom in Schritt b) bei einer Temperatur im Bereich von -10 °C bis 100 °C, bevorzugt im Bereich von 0 °C bis 90 °C, besonders bevorzugt im Bereich von 20 °C bis 80 °C kondensiert.

Der Druck bei der Kondensation des ersten Gasstroms in Schritt b) liegt beispielsweise im Bereich von 0,1 bar bis 1,1 bar, bevorzugt im Bereich von 0,3 bar bis 1 bar und insbesondere bevorzugt im Bereich von 0,4 bar bis 0,8 bar.

Bevorzugt erfolgt die Kondensation des ersten Gasstroms durch Einsaugen in zuvor bereits kondensierten und gekühlten ersten Gasstrom, wo der eingesaugte erste Gasstrom dann kondensiert. Ein Teilstrom des kondensierten ersten Gasstroms kann dann als flüssiger erster Strom abgetrennt werden, und erster Gasstrom erneut in den kondensierten und gekühlten ersten Gasstrom eingesaugt werden.

Bei der Kondensation geht der erste Gasstrom von der Gasphase in die flüssige Phase über unter Erhalt des flüssigen ersten Stroms.

Üblicherweise ist das im flüssigen ersten Strom enthaltene mindestens eine Alkyl(meth)acrylat dasselbe mindestens eine Alkyl(meth)acrylat, das im ersten Gasstrom enthalten war. Für das im flüssigen ersten Strom enthaltene mindestens eine Alkyl(meth)acrylat gelten daher die zuvor für das im ersten Gasstrom enthaltene mindestens eine Alkyl(meth)acrylat beschriebenen Ausführungen und Bevorzugungen entsprechend.

Üblicherweise ist der im flüssigen ersten Strom enthaltene mindestens eine weitere Alkylester derselbe mindestens eine weitere Alkylester, der im ersten Gasstrom enthalten war. Daher gelten für den im flüssigen ersten Strom enthaltenen mindestens einen weiteren Alkylester die zuvor beschrieben Ausführungen und Bevorzugungen wie für den im ersten Gasstrom enthaltenen mindestens einen weiteren Alkylester entsprechend.

Üblicherweise ist das im flüssigen ersten Strom enthaltene mindestens eine Benzolderivat dasselbe mindestens eine Benzolderivat, das im ersten Gasstrom enthalten war. Daher gelten für das im flüssigen ersten Strom enthaltene mindestens eine Benzolderivat die zuvor beschriebenen Ausführungen und Bevorzugungen wie für das im ersten Gasstrom enthaltene mindestens eine Benzolderivat entsprechend.

Der flüssige erste Strom kann zusätzlich die gegebenenfalls im ersten Gasstrom enthaltene mindestens eine weitere Komponente enthalten. Für die mindestens eine weitere Komponente gelten die zuvor beschriebenen Ausführungen und Bevorzugungen entsprechend.

Erfindungsgemäß bevorzugt wird nach Schritt a) und vor Schritt b) der erste Gasstrom destilliert. Dabei wird ein erster Kopfstrom, der das mindestens eine Alkyl(meth)acrylat und den mindestens einen weiteren Alkylester enthält, und ein erster Sumpfstrom, der das mindestens eine Benzolderivat und mindestens eine von dem mindestens einen Alkyl(meth)acrylat und dem mindestens einen weiteren Alkylester verschiedene Komponente enthält, erhalten. In dieser Ausführungsform wird dann der bei der Destillation erhaltene erste Kopfstrom in Schritt b) kondensiert. Der erste Kopfstrom kann Reste des mindestens einen Benzolderivats enthalten. Enthält der erste Kopfstrom Reste des mindestens einen Benzolderivats, so können diese im weiter unten beschriebenen Schritt e) ebenfalls abgetrennt werden. In einer Ausführungsform der Erfindung wird beispielsweise der mindestens eine weitere Alkylester in Gegenwart von Schwefelsäure hydrolysiert. Dann kann beispielsweise das mindestens eine Benzolderivat in Gegenwart dieser Schwefelsäure umgesetzt und so abgetrennt werden.

Bevorzugt ist daher auch ein Verfahren, bei dem der erste Gasstrom nach Schritt a) und vor Schritt b) destilliert wird unter Erhalt eines ersten Kopfstroms, der das mindestens eine Alkyl(meth)acrylat und den mindestens einen weiteren Alkylester enthält, und eines ersten Sumpfstroms, der mindestens eine von dem mindestens einen Alkyl(meth)acrylat und dem mindestens einen weiteren Alkylester verschiedene Komponente enthält, wobei der erste Kopfstrom in Schritt b) kondensiert wird.

Der erste Gasstrom kann kondensiert werden bevor er nach Schritt a) und vor Schritt b) destilliert wird. Verfahren zur Kondensation des ersten Gasstroms sind als solche bekannt und beispielsweise weiter oben beschrieben. Unter "Destillation des ersten Gasstroms" wird also im Rahmen der vorliegenden Erfindung nicht nur die Destillation des ersten Gasstroms in der Gasphase bezeichnet, sondern auch und insbesondere die Destillation des ersten Gasstroms nachdem er kondensiert wurde.

Die Destillation des ersten Gasstroms kann nach dem Fachmann bekannten Methoden und in dem Fachmann bekannten Reaktoren erfolgen. Beispielsweise kann der erste Gasstrom in eine Destillationskolonne und/oder in eine Rektifikationskolonne überführt und dort destilliert werden. Bei der Destillation des ersten Gasstroms werden Komponenten, die im ersten Gasstrom enthalten sind und die einen höheren Siedepunkt als das mindestens eine Alkyl(meth)acrylat aufweisen, im ersten Sumpfstrom erhalten, während Komponenten, die im ersten Gasstrom enthalten sind und denselben oder einen niedrigeren Siedepunkt als das mindestens eine Alkyl(meth)acrylat aufweisen, im ersten Kopfstrom erhalten werden.

Die Destillation kann beispielsweise bei einer Temperatur im Bereich von 40 °C bis 140 °C stattfinden. Bevorzugt liegt die Sumpftemperatur bei der Destillation im Bereich von 95 °C bis 130 °C, besonders bevorzugt im Bereich von 97 °C bis 126 °C.

Die Destillation kann beispielsweise bei einem Druck im Bereich von 10 mbar bis 250 mbar, bevorzugt im Bereich von 15 mbar bis 150 mbar stattfinden.

Bei der Destillation des ersten Gasstroms wird ein erster Kopfstrom erhalten. Der erste Kopfstrom enthält das mindestens eine Alkyl(meth)acrylat und den mindestens einen weiteren Alkylester, die bereits im ersten Gasstrom enthalten waren.

Der erste Kopfstrom kann darüber hinaus weitere Komponenten enthalten. Insbesondere enthält der erste Kopfstrom die im ersten Gasstrom enthaltene mindestens eine weitere Komponente, die einen niedrigeren oder genauso hohen Siedepunkt aufweist wie das mindestens eine Alkyl(meth)acrylat und der mindestens eine weitere Alkylester. Daher enthält der erste Kopfstrom üblicherweise mindestens eine weitere Komponente ausgewählt aus der Gruppe bestehend aus Methylpropionat, Ethylpropionat, Methylisobutyrat, Methylpivalat, Methyl-3-methoxyisobutyrat und Dicarbonsäurediestern.

Ebenso kann der erste Kopfstrom Reste des mindestens einen Benzolderivats enthalten.

Beispielsweise enthält der bei der Destillation erhaltene erste Kopfstrom 60 bis 99 Gew.-%, bevorzugt 80 bis 98 Gew.-%, des mindestens einen Alkyl(meth)acrylats, jeweils bezogen auf das Gesamtgewicht des ersten Kopfstroms.

Beispielsweise enthält der bei der Destillation erhaltene erste Kopfstrom 0,01 bis 5 Gew.-%, bevorzugt 0,1 bis 1 Gew.-%, des mindestens einen weiteren Alkylesters, jeweils bezogen auf das Gesamtgewicht des ersten Kopfstroms.

Beispielsweise enthält der bei der Destillation erhaltene erste Kopfstrom 0,01 bis 5 Gew.-%, bevorzugt 0,1 bis 3 Gew-%, der mindestens einen weiteren Komponente, jeweils bezogen auf das Gesamtgewicht des ersten Kopfstroms.

Außerdem wird ein erster Sumpfstrom erhalten. Unter einem "Sumpfstrom" wird im Rahmen der vorliegenden Erfindung nicht nur ein Sumpfprodukt verstanden, das kontinuierlich aus der Destillation, insbesondere aus dem Reaktor entfernt wird, sondern auch ein Sumpfprodukt, das, beispielsweise bei diskontinuierlicher Fahrweise als Destillationsrückstand im Reaktor verbleibt und erst zu einem späteren Zeitpunkt aus dem Reaktor entfernt wird.

Der erste Sumpfstrom enthält mindestens eine von dem mindestens einen Alkyl(meth)acrylat und dem mindestens einen weiteren Alkylester verschiedene Komponente. Diese Komponente ist üblicherweise mindestens eine der im ersten Gasstrom enthaltenen mindestens einen weiteren Komponenten. Insbesondere weist diese Komponente üblicherweise eine höhere Siedetemperatur auf als das im ersten Gasstrom enthaltene mindestens eine Alkyl(meth)acrylat und als der im ersten Gasstrom enthaltene mindestens eine weitere Alkylester. Beispielsweise ist diese Komponente ausgewählt aus der Gruppe bestehend aus schwefelhaltigen Verbindungen, Oligomeren und Dimeren. Für die schwefelhaltigen Verbindungen, die Oligomere und die Dimere gelten die zuvor für die in dem ersten Gasstrom enthaltenen schwefelhaltigen Verbindungen, die Oligomere und die Dimere beschriebenen Ausführungen und Bevorzugungen entsprechend.

Der erste Sumpfstrom enthält daher bevorzugt schwefelhaltige Verbindungen, Oligomere und/oder Dimere.

Darüber hinaus enthält der erste Sumpfstrom das mindestens eine Benzolderivat.

In Schritt c) des erfindungsgemäßen Verfahrens wird der in Schritt b) erhaltene flüssige erste Strom mit einem weiteren Strom, der Alkyl(meth)acrylat enthält, gemischt unter Erhalt eines Mischstroms. Der weitere Strom ist Teil eines Alkyl(meth)acrylat-Herstellverfahrens. Der Mischstrom umfasst den flüssigen ersten Strom und den weiteren Strom.

Beispielsweise werden bis zu 20 Gew.-%, bevorzugt bis zu 10 Gew.-%, besonders bevorzugt bis zu 5 Gew.-% des flüssigen ersten Stroms mit dem weiteren Strom gemischt, bezogen auf das Gesamtgewicht des Mischstroms.

Der weitere Strom, mit dem der flüssige erste Strom gemischt wird, enthält Alkyl(meth)acrylat. Der weitere Strom kann genau ein Alkyl(meth)acrylat enthalten oder eine Mischung aus zwei oder mehreren Alkyl(meth)acrylaten. Genau ein Alkyl(meth)acrylat ist bevorzugt. Für das im weiteren Strom enthaltene Alkyl(meth)acrylat gelten die zuvor für das im ersten Gasstrom und im flüssigen ersten Strom enthaltene mindestens eine Alkyl(meth)acrylat beschriebenen Ausführungen und Bevorzugungen entsprechend. Das Alkyl(meth)acrylat ist beispielsweise ausgewählt aus der Gruppe bestehend aus C₁-C₄-Alkyl(meth)acrylaten. Insbesondere bevorzugt ist das im weiteren Strom enthaltene Alkyl(meth)acrylat Methyl(meth)acrylat.

Das im flüssigen ersten Strom enthaltene mindestens eine Alkyl(meth)acrylat umfasst bevorzugt dasselbe Alkyl(meth)acrylat wie der weitere Strom enthält.

Es ist erfindungsgemäß bevorzugt, dass, bezogen auf das Gesamtgewicht des im flüssigen ersten Stroms enthaltenen mindestens eine Alkyl(meth)acrylats, mindestens 80 Gew.-%, bevorzugt mindestens 90 Gew.-% des im flüssigen ersten Stroms enthaltenen mindestens einen Alkyl(meth)acrylats dasselbe Alkyl(meth)acrylat ist wie das im weiteren Strom enthaltene Alkyl(meth)acrylat.

Der weitere Strom ist Teil eines Alkyl(meth)acrylat-Herstellverfahrens, insbesondere eines Methyl(meth)acrylat-Herstellverfahrens. Der weitere Strom kann beispielsweise Teil eines C₃-Verfahrens sein. Ebenso ist es möglich, dass der weiter Strom Teil eines C₂-Verfahrens ist. Darüber hinaus kann der weitere Strom Teil eines C₄-Verfahrens sein. Bevorzugt ist der weitere Strom Teil eines Cs-Verfahrens.

Erfindungsgemäß bevorzugt ist daher auch ein Verfahren, bei dem das Alkyl(meth)acrylat-Herstellverfahren, dessen Teil der weitere Strom in Schritt c) ist, ein Methylmethacrylat-Herstellverfahren ist, das ausgewählt ist aus der Gruppe bestehend aus einem C₂-, einem C₃- und einem C₄-Verfahren.

Der weitere Strom kann noch zusätzliche Komponenten enthalten. Beispielsweise enthält der weitere Strom zumindest eine zusätzliche Komponente ausgewählt aus der Gruppe bestehend aus Methylpropionat und Methylisobutyrat.

Ist der weitere Strom Teil eines C₂-Verfahrens, so enthält der weitere Strom zumindest eine zusätzliche Komponente ausgewählt aus der Gruppe bestehend aus Methylpropionat, Methylisobutyrat, Isobutyraldehyd-Dimethylacetal, Methacrolein-Dimethylacetal, Isobutanal, Isobutenol, 2-Methoxyaceton, 2-Methoxyisopropanol und Methyl(meth)acrylat-Dimeren.

Ist der weitere Strom Teil eines Cs-Verfahrens (ACH-Verfahren), so enthält der weitere Strom zumindest eine zusätzliche Komponente ausgewählt aus der Gruppe bestehend aus Methylisopropionat, Methylisobutyrat, Methyl-3-methoxyisobutyrat, Diacetyl, Methacrylnitril, Hydroxyisobuttersäuremethylester, Methylformiat, Ameisensäure und Methacrylsäure.

Ist der weitere Strom Teil eines C₄-Verfahrens, so enthält der weitere Strom zumindest eine zusätzliche Komponente ausgewählt aus der Gruppe bestehend aus Methylpropionat, Methylisobutyrat, Isobutyraldehyd-Dimethylacetal, Isobutanol, Aceton, Brenztraubensäuremethylester, Diacetyl, Ethylmethacrylat, Isopropylmethacrylat, Methyl-tert-butylether, Dimethylfuran und Methacrylsäure.

Der flüssige erste Strom kann nach dem Fachmann bekannten Methoden mit dem weiteren Strom gemischt werden. Beispielsweise und erfindungsgemäß bevorzugt wird der flüssige erste Strom in eine Anlage zur Herstellung eines Alkyl(meth)acrylats eingespeist, die den weiteren Strom enthält. Der erhaltene Mischstrom ist dann vorzugsweise ebenfalls in der Anlage zur Herstellung eines Alkyl(meth)acrylats enthalten. Der Mischstrom ist dann als vorzugsweise ebenfalls Teil des Alkyl(meth)acrylat Herstellverfahrens.

Bevorzugt ist daher also auch ein Verfahren, bei dem er in Schritt c) erhaltene Mischstrom Teil des Alkyl(meth)acrylat Herstellverfahrens ist.

Der Mischstrom umfasst den flüssigen ersten Strom und den weiteren Strom. Der Mischstrom umfasst daher üblicherweise dieselben Komponenten, die im flüssigen ersten Strom und in dem weiteren Strom enthalten waren. Daher umfasst der Mischstrom üblicherweise das mindestens eine Alkyl(meth)acrylat, den mindestens einen weiteren Alkylester, das Alkyl(meth)acrylat sowie gegebenenfalls die zusätzlichen Komponenten, das mindestens eine Benzolderivat und/oder die mindestens eine weitere Komponente.

In Schritt d) wird der in Schritt c) erhaltene Mischstrom unter Erhalt eines Sumpfstroms und eines Kopfstroms destilliert. Der Sumpfstrom enthält das Alkyl(meth)acrylat, der Kopfstrom enthält den mindestens einen weiteren Alkylester.

Die Destillation des Mischstroms in Schritt d) kann nach dem Fachmann bekannten Methoden und in dem Fachmann bekannten Reaktoren erfolgen. Beispielsweise kann der Mischstrom in eine erste Destillationskolonne und/oder eine erste Rektifikationskolonne überführt werden. Bevorzugt ist die erste Destillationskolonne und/oder die erste Rektifikationskolonne Teil des Alkyl(meth)acrylat-Herstellverfahrens.

Erfindungsgemäß wird bei der Destillation in Schritt d) ein Sumpfstrom erhalten, der das Alkyl(meth)acrylat enthält, und ein Kopfstrom, der den mindestens einen weiteren Alkylester enthält.

Bei der Destillation in Schritt d) werden üblicherweise Komponenten, die in dem Mischstrom enthalten sind und einen niedrigeren Siedepunkt aufweisen als das mindestens eine Alkyl(meth)acrylat, in dem Kopfstrom erhalten. Im Sumpfstrom werden Komponenten, die in dem Mischstrom enthalten sind und einen genauso hohen oder höheren Siedepunkt aufweisen wie das mindestens eine Alkyl(meth)acrylat. Daher wird die Destillation in Schritt d) auch als Niedrigsiederdestillation bezeichnet. Sie findet üblicherweise in einer Niedrigsiederkolonne des Alkyl(meth)acrylat-Herstellverfahrens statt.

Der Kopfstrom enthält den mindestens einen weiteren Alkylester. Darüber hinaus kann der Kopfstrom Reste des mindestens einen Alkyl(meth)acrylats enthalten.

Unter "Resten des Alkyl(meth)acrylats" werden im Rahmen der vorliegenden Erfindung beispielsweise im Bereich von 25 Gew.-% bis 30 Gew.-% des mindestens einen Alkyl(meth)acrylats verstanden, bezogen auf das Gesamtgewicht des Kopfstroms.

Der Kopfstrom kann beispielsweise in das Alkyl(meth)acrylat-Herstellverfahren recycliert werden.

Der Sumpfstrom enthält das mindestens eine Alkyl(meth)acrylat. Der Sumpfstrom kann darüber hinaus zusätzlich Reste des mindestens einen weiteren Alkylesters enthalten.

Unter "Reste des mindestens einen weiteren Alkylesters" werden beispielsweise im Bereich von 0,1 Gew.-ppm bis 1 Gew.-% des mindestens einen weiteren Alkylesters, bezogen auf das Gesamtgewicht des im Mischstrom enthaltenen mindestens einen weiteren Alkylesters verstanden.

Unter "Reste des mindestens einen weiteren Alkylesters" wird im Rahmen auch verstanden, dass der Mischstrom eine Mischung aus zwei oder mehreren weiteren Alkylestern enthält, wobei mindestens einer der weiteren Alkylestern einen niedrigeren Siedepunkt als das mindestens eine Alkyl(meth)acrylat und mindestens einer der weiteren Alkylester einen höheren Siedepunkt als das mindestens eine Alkyl(meth)acrylat aufweist und der Kopfstrom dann den weiteren Alkylester mit dem niedrigen Siedepunkt enthält, während der Sumpfstrom den weiteren Alkylester mit dem höheren Siedepunkt enthält.

Beispielsweise kann der Mischstrom als den mindestens einen weiteren Alkylester Methylpropionat und Methyl-3-Methoxyisobutyrat enthalten. Dann kann der bei der Destillation in Schritt d) erhaltene Kopfstrom Methylpropionat als den mindestens einen weiteren Alkylester enthalten, und der in Schritt d) erhaltene Sumpfstrom beispielsweise Methyl-3-Methoxyisobutyrat als Reste des mindestens einen weiteren Alkylesters.

Bevorzugt enthält der Sumpfstrom zusätzlich Reste des mindestens einen weiteren Alkylesters und im Anschluss an Schritt d) wird der folgende Schritt d1) durchgeführt:
d1) Destillation des in Schritt d) erhaltenen Sumpfstroms unter Erhalt eines zweiten Kopfstroms, der Alkyl(meth)acrylat enthält, und eines zweiten Sumpfstroms, der den mindestens einen weiteren Alkylester enthält.

Erfindungsgemäß bevorzugt ist daher auch ein Verfahren, bei dem der in Schritt d) erhaltene Sumpfstrom zusätzlich Reste des mindestens einen weiteren Alkylesters enthält und dass im Anschluss an Schritt d) der folgende Schritt d1) durchgeführt wird:
d1) Destillation des in Schritt d) erhaltenen Sumpfstroms unter Erhalt eines zweiten Kopfstroms, der Alkyl(meth)acrylat enthält, und eines zweiten Sumpfstroms, der den mindestens einen weiteren Alkylester enthält.

Die Destillation in Schritt d1) kann nach dem Fachmann bekannten Methoden in dem Fachmann bekannten Reaktoren erfolgen. Beispielsweise kann der in Schritt d) erhaltene Sumpfstrom in eine zweite Destillationskolonne und/oder eine zweite Rektifikationskolonne überführt werden. Bevorzugt ist die zweite Destillationskolonne und/oder die zweite Rektifikationskolonne Teil des Alkyl(meth)acrylat-Herstellverfahrens.

Bei der Destillation in Schritt d1) werden üblicherweise Komponenten, die im Sumpfstrom enthalten sind und einen höheren Siedepunkt aufweisen als das mindestens eine Alkyl(meth)acrylat im Sumpfstrom erhalten. Im Kopfstrom werden Komponenten erhalten, die denselben oder einen niedrigeren Siedepunkt aufweisen wie das mindestens eine Alkyl(meth)acrylat. Daher wird die Destillation in Schritt d1) auch als Hochsiederdestillation bezeichnet. Sie findet üblicherweise in einer Hochsiederkolonne des Alkyl(meth)acrylat-Herstellverfahrens statt.

Erfindungsgemäß wird bei der Destillation in Schritt d1) ein zweiter Kopfstrom, der Alkyl(meth)acrylat enthält, und ein zweiter Sumpfstrom, der den mindestens einen weiteren Alkylester enthält, erhalten.

Der zweite Sumpfstrom kann darüber hinaus weitere Komponenten enthalten, wie beispielsweise die im Mischstrom gegebenenfalls enthaltene zusätzliche Komponente und/oder die mindestens eine weitere Komponente. Insbesondere kann der zweite Sumpfstrom als weitere Komponente schwefelhaltige Verbindungen enthalten.

Der zweite Sumpfstrom kann weiter umgesetzt werden. Beispielsweise kann der zweite Sumpfstrom mit einem säurehaltigen Strom umgesetzt werden.

Beispielsweise kann der zweite Sumpfstrom mit einem Ammoniumhydrogensulfat-haltigen Strom umgesetzt werden. In diesem Ammoniumhydrogensulfat-haltigen Strom können dann die schwefelhaltigen Verbindungen mittels Partialoxidation umgesetzt werden.

Bevorzugt ist daher auch ein Verfahren, bei dem der zweite Sumpfstrom mit einem Ammoniumhydrogensulfat-haltigen Strom umgesetzt wird unter Erhalt einer schwefelsäurehaltigen Mischung.

Es ist insbesondere bevorzugt, dass der Ammoniumhydrogensulfat-haltige Strom aus einem C₃-Verfahren zur Herstellung von Methyl(meth)acrylat stammt oder Teil eines Cs-Verfahrens zur Herstellung von Methyl(meth)acrylat ist.

Der zweite Sumpfstrom kann zusätzlich oder alternativ, bevorzugt alternativ, mit einem (Meth)acrylsäure enthaltenden Strom vermischt werden.

Bevorzugt ist daher auch ein Verfahren, bei dem der zweite Sumpfstrom mit einem (Meth)acrylsäure enthaltenden Strom vermischt wird.

Es ist erfindungsgemäß bevorzugt, dass der (Meth)acrylsäure enthaltende Strom aus einem C₄-Verfahren zur Herstellung von Methyl(meth)acrylat stammt oder Teil eines C₄-Verfahrens zur Herstellung von Methyl(meth)acrylat ist.

Der zweite Sumpfstrom kann zusätzlich oder alternativ, bevorzugt alternativ, mit einem 3-Methoxyisobuttersäuremethylester- und/oder Methylpropionat-haltigen Strom in vermischt werden.

Es ist erfindungsgemäß bevorzugt, dass der Methoxyisobuttersäuremethylester- und/oder Methylpropionat-haltige Strom aus einem C₂-Verfahren zur Herstellung von Methyl(meth)acrylat stammt oder Teil eines C₂-Verfahrens zur Herstellung von Methyl(meth)acrylat ist. Das C₂-Verfahren ist dem Fachmann als solches bekannt.

In Schritt e) des erfindungsgemäßen Verfahrens wird der in Schritt d) erhaltene Kopfstrom zumindest teilweise hydrolysiert. Dabei hydrolysiert der in dem Kopfstrom enthaltene mindestens eine weitere Alkylester zumindest teilweise unter Erhalt einer Alkylsäure.

Der in Schritt d) erhaltene Kopfstrom kann nach dem Fachmann bekannten Methoden hydrolysiert werden. Beispielsweise kann die Hydrolyse über einen sauren Ionenaustauscher erfolgen. Ebenso ist es möglich, dass die Hydrolyse mit einer Säure, beispielsweise mit Schwefelsäure erfolgt.

Zur zumindest teilweisen Hydrolyse des in Schritt d) erhaltenen Kopfstroms kann der Kopfstrom aus dem Alkyl(meth)acrylat-Herstellverfahren entfernt werden. Ebenso ist es möglich und erfindungsgemäß bevorzugt, dass die zumindest teilweise Hydrolyse in Schritt e) innerhalb des Alkyl(meth)acrylat-Herstellverfahrens erfolgt, dessen Teil der weitere Strom ist.

Erfindungsgemäß bevorzugt ist daher auch ein Verfahren, bei dem die zumindest teilweise Hydrolyse in Schritt e) innerhalb des Alkyl(meth)acrylat-Herstellverfahrens stattfindet, dessen Teil der weitere Strom ist.

Umfasst das Alkyl(meth)acrylat-Herstellverfahren beispielsweise eine Veresterungsstufe, so ist es erfindungsgemäß bevorzugt, dass die Hydrolyse in Schritt e) in Gegenwart einer Säure im Alkyl(meth)acrylat-Herstellverfahren stattfindet.

Bevorzugt ist daher auch ein Verfahren, bei die zumindest teilweise Hydrolyse in Schritt e) innerhalb des Alkyl(meth)acrylat-Herstellverfahrens in Gegenwart eines Säure im Alkyl(meth)acrylat-Herstellverfahren stattfindet.

Beispielsweise ist das Alkyl(meth)acrylat-Herstellverfahren ein C₃-Verfahren. Dann kann die Hydrolyse in Schritt e) beispielsweise im Bereich der Amidierung von Acetoncyanhydrin erfolgen. Die bei der Hydrolyse des mindestens einen weiteren Alkylesters erhaltene Alkylsäure kann dann aus dem Alkyl(meth)acrylat-Herstellverfahren nach dem Fachmann bekannten Methoden abgetrennt werden.

Vorzugsweise wird der in Schritt d) erhaltene Kopfstrom kondensiert und einer Phasentrennung unterzogen. In der Phasentrennung werden eine organische Phase und eine wässrige Phase erhalten. Der mindestens eine weitere Alkylester hat vorzugsweise eine höhere Löslichkeit in der wässrigen Phase als das Alkyl(meth)acrylat. In der wässrigen Phase wird der mindestens eine weitere Alkylester gemäß Schritt e) hydrolysiert.

In Schritt d) des erfindungsgemäßen Verfahrens wird das Alkyl(meth)acrylat erhalten. Insbesondere wird das Alkyl(meth)acrylat im zweiten Kopfstrom in Schritt d1) erhalten. Das erfindungsgemäß erhaltene Alkyl(meth)acrylat enthält im Wesentlichen das Alkyl(meth)acrylat, das im Alkyl(meth)acrylat-Herstellverfahren dessen Teil der weitere Strom ist erhalten wird.

Erfindungsgemäß enthält der flüssige erste Strom das mindestens eine Alkyl(meth)acrylat, mindestens einen weiteren Alkylester und mindestens ein Benzolderivat sowie gegebenenfalls mindestens eine weitere Komponente. Das erfindungsgemäße Verfahren ermöglicht die Isolierung von Alkyl(meth)acrylat, das im Wesentlichen frei ist von diesen übrigen im flüssigen ersten Strom enthaltenen Komponenten.

Das im erfindungsgemäßen Verfahren erhaltene Alkyl(meth)acrylat besteht daher im Wesentlichen aus genau einem Alkyl(meth)acrylat. Für das Alkyl(meth)acrylat gelten die zuvor beschriebenen

Ausführungen und Bevorzugungen. Besonders bevorzugt ist das erhaltene Alkyl(meth)acrylat Methyl(meth)acrylat, das höchstens 0,1 Gew.-%, bevorzugt höchstens 0,01 Gew.-% weiterer Komponenten und/oder des mindestens einen Alkylester enthält.

Das erfindungsgemäß isolierte Alkyl(meth)acrylat kann beispielsweise in dem Fachmann bekannten Verfahren zur Herstellung von Alkyl(meth)acrylat-Oligomeren und/oder Alkyl(meth)acrylat-Polymeren durch Polymerisation eingesetzt werden.

Gegenstand der vorliegenden Erfindung ist daher auch ein Verfahren zur Herstellung von Alkyl(meth)acrylat-Oligomeren und/oder Alkyl(meth)acrylat-Polymeren durch Polymerisation eines in einem erfindungsgemäßen Verfahren isolierten Alkyl(meth)acrylats.

Das erfindungsgemäß isolierte Alkyl(meth)acrylat kann beispielsweise zur Herstellung von (Meth)acrylsäure durch Hydrolyse des Alkyl(meth)acrylats verwendet werden.

Gegenstand der vorliegenden Erfindung ist daher auch ein Verfahren zur Herstellung von (Meth)acrylsäure durch Hydrolyse eines in einem erfindungsgemäßen Verfahren isolierten Alkyl(meth)acrylats.

Verfahren zur Herstellung von (Meth)acrylsäure aus Alkyl(meth)acrylaten sind dem Fachmann als solche bekannt.

Weiterhin kann das erfindungsgemäß isolierte Alkyl(meth)acrylat zur Herstellung von Alkyl(meth)acrylat-Monomeren durch Umesterung des erfindungsgemäßen Alkyl(meth)acrylats verwendet werden.

Geeignete Umesterungsverfahren sind dem Fachmann als solche bekannt.

Gegenstand der vorliegenden Erfindung ist daher auch ein Verfahren zur Herstellung von Alkyl(meth)acrylat-Monomeren durch Umesterung eines in einem erfindungsgemäßen Verfahren isolierten Alkyl(meth)acrylats.

Ein weiterer Gegenstand der vorliegenden Erfindung ist ein Verfahren zur Isolierung von Alkyl(meth)acrylaten umfassend die folgenden Schritte c1) bis e):
c1) Mischen eines flüssigen ersten Stroms, der mindestens ein Alkyl(meth)acrylat, mindestens einen weiteren Alkylester und mindestens ein Benzolderivat enthält, wobei der flüssige erste Strom durch thermische Spaltung von mindestens einer Polymerzusammensetzung erhältlich ist, mit einem weiteren Strom, wobei der weitere Strom Teil eines Alkyl(meth)acrylat-Herstellverfahrens ist, unter Erhalt eines Mischstroms, der den flüssigen ersten Strom und den weiteren Strom umfasst,
d) Destillation des in Schritt c) erhaltenen Mischstroms unter Erhalt eines Sumpfstroms, der Alkyl(meth)acrylat enthält, und eines Kopfstroms, der den mindestens einen weiteren Alkylester enthält,
e) zumindest teilweise Hydrolyse des in Schritt d) erhaltenen Kopfstroms, wobei der mindestens eine weitere Alkylester zumindest teilweise hydrolysiert unter Erhalt einer Alkylsäure.

In Schritt c1) wird ein flüssiger erster Strom mit einem weiteren Strom gemischt. der flüssige erste Strom ist erhältlich durch thermische Spaltung von mindestens einer Polymerzusammensetzung. Für die Polymerzusammensetzung gelten die zuvor beschriebenen Ausführungen und Bevorzugungen.

Bevorzugt ist der flüssige erste Strom daher erhältlich durch die folgenden Schritte a) und b):
a) thermische Spaltung von mindestens einer Polymerzusammensetzung, die mindestens ein Poly(alkyl(meth)acrylat) enthält, unter Erhalt eines ersten Gasstroms, der mindestens ein Alkyl(meth)acrylat, mindestens einen weiteren Alkylester und mindestens ein Benzolderivat enthält,
b) Kondensation des in Schritt a) erhaltenen ersten Gasstroms unter Erhalt eines flüssigen ersten Stroms, der das mindestens eine Alkyl(meth)acrylat, den mindestens einen weiteren Alkylester und mindestens ein Benzolderivat enthält.

Für die Schritte a) und b) gelten die zuvor beschriebenen Ausführungen und Bevorzugungen entsprechend. Für den Schritt c1) gelten die zuvor für Schritt c) beschriebenen Ausführungen und Bevorzugungen entsprechend.

Für die Schritte d) und e) gelten die zuvor beschriebene Ausführungen entsprechend.

## Patentansprüche

1. Verfahren zur Isolierung von Alkyl(meth)acrylaten umfassend die folgenden Schritte a) bis e):
a) thermische Spaltung von mindestens einer Polymerzusammensetzung, die mindestens ein Poly(alkyl(meth)acrylat) enthält, unter Erhalt eines ersten Gasstroms, der mindestens ein Alkyl(meth)acrylat, mindestens einen weiteren Alkylester und mindestens ein Benzolderivat enthält,
b) Kondensation des in Schritt a) erhaltenen ersten Gasstroms unter Erhalt eines flüssigen ersten Stroms, der das mindestens eine Alkyl(meth)acrylat, den mindestens einen weiteren Alkylester und mindestens ein Benzolderivat enthält,
c) Mischen des in Schritt b) erhaltenen flüssigen ersten Stroms mit einem weiteren Strom, der Alkyl(meth)acrylat enthält, wobei der weitere Strom Teil eines Alkyl(meth)acrylat-Herstellverfahrens ist, unter Erhalt eines Mischstroms, der den flüssigen ersten Strom und den weiteren Strom umfasst,
d) Destillation des in Schritt c) erhaltenen Mischstroms unter Erhalt eines Sumpfstroms, der Alkyl(meth)acrylat enthält, und eines Kopfstroms, der den mindestens einen weiteren Alkylester enthält,
e) zumindest teilweise Hydrolyse des in Schritt d) erhaltenen Kopfstroms, wobei der mindestens eine weitere Alkylester zumindest teilweise hydrolysiert unter Erhalt einer Alkylsäure.

2. Verfahren gemäß Anspruch 1, **dadurch gekennzeichnet, dass** die Polymerzusammensetzung in Schritt a) zumindest eine weitere Komponente, ausgewählt aus der Gruppe bestehend aus von Poly(alkyl(meth)acrylat) verschiedenen Polymeren, Pigmenten, Farbstoffen, Füllstoffen, Hilfsstoffen, Reglern, Initiatoren, Schlagzähmachern, Trennmitteln und UV-Additiven, enthält.

3. Verfahren gemäß Anspruch 1 oder 2, **dadurch gekennzeichnet, dass** das in dem ersten Gasstrom in Schritt a) enthaltene mindestens eine Alkyl(meth)acrylat ausgewählt ist aus der Gruppe bestehend aus C₁- bis C₄-Alkyl(meth)acrylaten.

4. Verfahren gemäß einem der Ansprüche 1 bis 3, **dadurch gekennzeichnet, dass** der mindestens eine weitere Alkylester ausgewählt ist aus der Gruppe bestehend aus Methylpropionat, Methylisobutyrat, Methylpivalat, Methyl-3-methoxyisobutyrat und Dicarbonsäurediestern.

5. Verfahren gemäß einem der Ansprüche 1 bis 4, **dadurch gekennzeichnet, dass** der erste Gasstrom mindestens eine weitere Komponente, ausgewählt aus der Gruppe bestehend aus(Meth)acrylsäure, schwefelhaltigen Verbindungen, Oligomeren und Dimeren, enthält.

6. Verfahren gemäß einem der Ansprüche 1 bis 5, **dadurch gekennzeichnet, dass** der erste Gasstrom nach Schritt a) und vor Schritt b) destilliert wird unter Erhalt eines ersten Kopfstroms, der das mindestens eine Alkyl(meth)acrylat und den mindestens einen weiteren Alkylester enthält, und eines ersten Sumpfstroms, der mindestens eine von dem mindestens einen Alkyl(meth)acrylat und dem mindestens einen weiteren Alkylester verschiedene Komponente enthält, wobei der erste Kopfstrom in Schritt b) kondensiert wird.

7. Verfahren gemäß einem der Ansprüche 1 bis 6, **dadurch gekennzeichnet, dass** der in Schritt d) erhaltene Sumpfstrom zusätzlich Reste des mindestens einen weiteren Alkylesters enthält und dass im Anschluss an Schritt d) der folgende Schritt d1) durchgeführt wird:
d1) Destillation des in Schritt d) erhaltenen Sumpfstroms unter Erhalt eines zweiten Kopfstroms, der Alkyl(meth)acrylat enthält, und eines zweiten Sumpfstroms, der den mindestens einen weiteren Alkylester enthält.

8. Verfahren gemäß Anspruch 7, **dadurch gekennzeichnet, dass** der zweite Sumpfstrom mit einem Ammoniumhydrogensulfat-haltigen Strom umgesetzt wird unter Erhalt einer schwefelsäurehaltigen Mischung.

9. Verfahren gemäß Anspruch 7, **dadurch gekennzeichnet, dass** der zweite Sumpfstrom mit einem (Meth)acrylsäure enthaltenden Strom vermischt wird.

10. Verfahren gemäß einem der Ansprüche 1 bis 9, **dadurch gekennzeichnet, dass** das Alkyl(meth)acrylat-Herstellverfahren, dessen Teil der weitere Strom in Schritt c) ist, ein Methylmethacrylat-Herstellverfahren ist, das ausgewählt ist aus der Gruppe bestehend aus einem C₂-, einem C₃- und einem C₄-Verfahren.

11. Verfahren gemäß einem der Ansprüche 1 bis 10, **dadurch gekennzeichnet, dass** die zumindest teilweise Hydrolyse in Schritt e) innerhalb des Alkyl(meth)acrylat-Herstellverfahrens stattfindet, dessen Teil der weitere Strom ist.

12. Verfahren gemäß Anspruch 11, **dadurch gekennzeichnet, dass** die zumindest teilweise Hydrolyse in Schritt e) innerhalb des Alkyl(meth)acrylat-Herstellverfahrens in Gegenwart eines Säure im Alkyl(meth)acrylat-Herstellverfahren stattfindet.

13. Verfahren zur Herstellung von Alkyl(meth)acrylat-Oligomeren und/oder Alkyl(meth)acrylat-Polymeren durch Polymerisation eines in einem Verfahren gemäß einem der Ansprüche 1 bis 12 isolierten Alkyl(meth)acrylats.

14. Verfahren zur Herstellung von (Meth)acrylsäure durch Hydrolyse eines in einem Verfahren gemäß einem der Ansprüche 1 bis 12 isolierten Alkyl(meth)acrylats.

15. Verfahren zur Herstellung von Alkyl(meth)acrylat-Monomeren durch Umesterung eines in einem Verfahren gemäß einem der Ansprüche 1 bis 12 isolierten Alkyl(meth)acrylats.
